# EUROPEAN PATENT APPLICATION

(11) **EP 4 458 363 A1**
(43) Date of publication of application: **06.11.2024**
(21) Application number: 23382409.3
(22) Date of filing: 03.05.2023
(51) Int. Cl.: A61K 35/16, A61K 35/19, A61K 35/28, A61K 35/32, A61K 9/00, A61P 17/00

(54) **PROCESS OF MANUFACTURING A COMPOSITION OF PLASMA AND USES THEREOF**

(71) Applicant: Tecbiocel S.L., 20017 Donostia-San Sebastián (ES)
(72) Inventor: GONZÁLEZ POZAS, Federico, 2009 Donostia-San Sebastián (ES); FERNÁNDEZ SAN MIGUEL, Ainhoa, 2009 Donostia-San Sebastián (ES)
(74) Representative: Hoffmann Eitle

(57) **Abstract**

The present invention refers to a method for obtaining a composition of plasma rich in growth factors, particularly suitable for allogenic use, comprising the steps of:
a) mixing platelet-rich plasma (hereinafter referred to as "PRP") obtained by centrifugation of an anticoagulated sample of whole blood, Bone marrow, Menstrual fluid, or Umbilical cord blood, with a formulation agent (FA) at a ratio PRP:FA of from 1:1 to 1:10, wherein the formulation agent is selected from a pharmaceutically acceptable excipient or a pharmaceutically acceptable additive;
b) activating the platelet rich plasma (PRP) resultant from step (a);
c) centrifuging the product resultant from step (b) in the range of from 100g to 6000g, for a time of between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

## Description

### FIELD OF THE INVENTION

The invention relates to method of manufacturing a composition of blood plasma especially useful for allogenic use.

### BACKGROUND OF THE INVENTION

Platelet-rich plasma (PRP) is frequently used in the treatment of acute and chronic wounds. One of the major problems concerning the use of PRP is the absence of a well-characterized and standardized product, which leads to a high variety in study outcomes. Therefore, more studies on the composition and standardization of PRP, such as in wound healing, are needed.

In addition, allogeneic PC-plasma (platelet compound plasma) has beneficial effects on various aspects of many diseases such as wound healing and is superior to plasma or platelets alone for such use.

The present invention provides for a method to manufacture well-characterized and standardized compositions of plasma, preferably blood plasma rich in growth factors, especially suitable for allogeneic use.

### SUMMARY OF THE INVENTION

The present invention particularly refers to a method for obtaining a composition of plasma rich in growth factors, particularly suitable for allogenic use, comprising the steps of:
a) mixing platelet-rich plasma (hereinafter referred to as "PRP") obtained by centrifugation of an anticoagulated sample of whole blood, bone marrow, menstrual fluid, or umbilical cord blood, with a formulation agent (FA) at a ratio PRP:FA of from 1:1 to 1:10, wherein the formulation agent is selected from a pharmaceutically acceptable excipient or a pharmaceutically acceptable additive;
b) activating the platelet rich plasma (PRP) resultant from step (a);
c) centrifuging the product resultant from step (b) in a range of from 100g to 6000g, for a time between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

A composition of plasma rich in growth factors obtained or obtainable by the method above and uses of this composition thereof.

Other features and advantages of the present invention will become apparent from the following detailed description examples and figures. It should be understood, however, that the detailed description and the specific examples while indicating preferred embodiments of the invention are given by way of illustration only, since various changes and modifications within the spirit and scope of the invention will become apparent to those skilled in the art from this detailed description.

### BRIEF DESCRIPTION OF THE DRAWINGS

**Fig 1****. Coombs test of example 1.** Comparative between "Auto PRGF", which corresponds to autologous PRGF; "Allo PRGF", which corresponds to allogeneic PRGF and "O+ blood", which corresponds to the donor's blood group (O+)

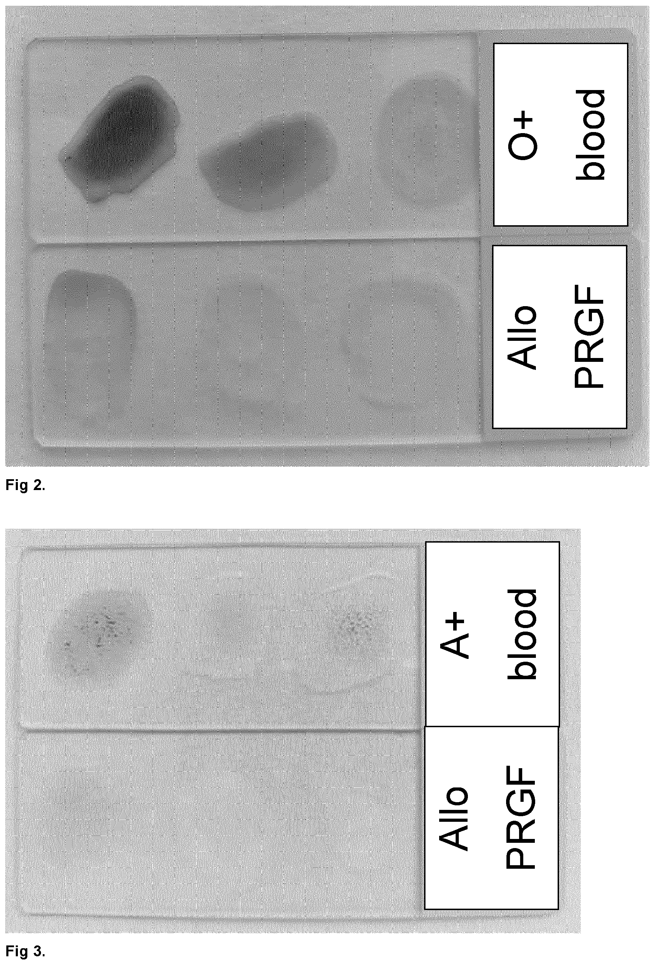

**Fig 2****. Combs test of example 2.** Comparative between "O+ blood", which corresponds to the donor's blood group (O+); and "Allo PRGF", which corresponds to allogeneic PRGF.
**Fig 3****. Coombs test of example 3.** Comparative between "A+ blood", which corresponds to the donor's blood group (A+); and "Allo PRGF", which corresponds to allogeneic PRGF.
**Fig 4****. Scheme of the manufacturing process of the allogeneic PRGF.**
**Fig 5****. Coombs test of example 4.** Coombs test of an autologous sample "A- blood) and allogeneic PRGF sample. Comparison test of the simples analyzing different parameters included in sample 4, such as temperature (50°C, 56°C and 65°C) and incubation period (30, 60 and 90 minutes)
**Fig 6****. Flow cytometry of example 4.** Flow cytometry analysis to identify CD4+ and CD8+ cells in a blood sample. The left upper plot refers to the lymphocytes isolated from the blood sample without any staining, and the right upper plot refers to the lymphocytes isolated from the blood sample stained with CD4 and CD8. Tables below refers to the statistical analysis of the markers involved in each sample.
**Fig 7****. Flow cytometry of example 4.** Flow cytometry analysis to identify CD4+ and CD8+ cells in autologous and allogeneic PRGF samples. The left upper plot refers to the "autologous PRGF", and the right upper plot refers to the "allogeneic PRGF", both stained with CD4 and CD8. Tables below refers to the statistical analysis of the markers involved in each sample.
**Fig 8****. CH50 assay of PRGF autologous vs. PRGF allogeneic (n=3).** Measures in U/ml. ** p-value<0.01.

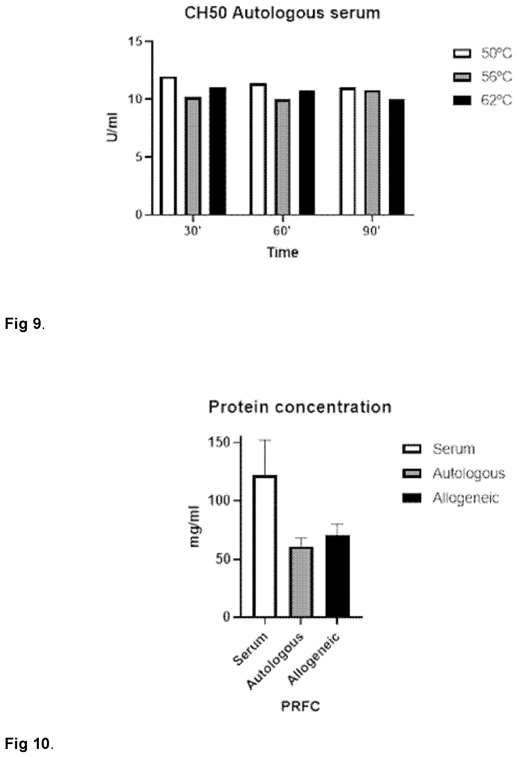

**Fig 9****. CH50 assay of PRGF allogeneic diluted 1:2 in human serum** at different temperatures (50°C, 56°C and 62°C) and incubation periods (30, 60 and 90 minutes).
**Fig 10****. Bradford protein assay of human human serum, autologous PRGF and allogeneic PRGF (n=3).** Protein quantification is shown in mg/ml.

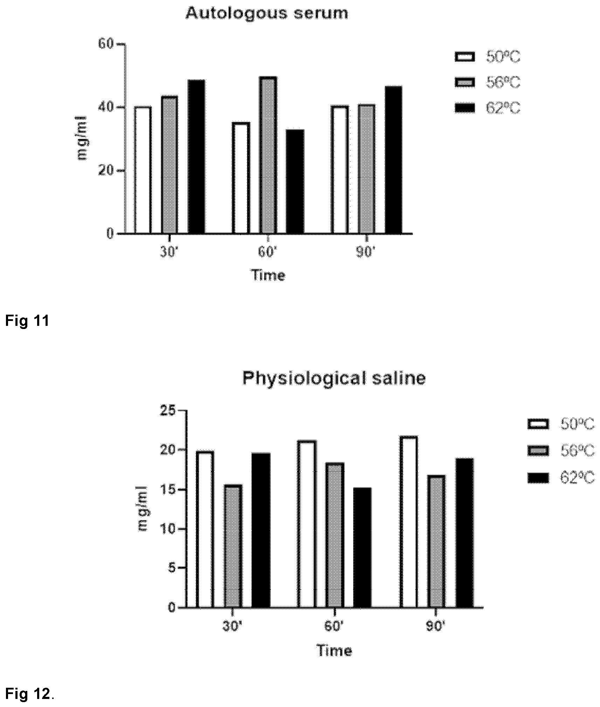

**Fig 11** **Bradford protein assay of allogeneic PRGF diluted in human serum (1:2) (example 4).** Protein quantification (mg/ml) at different temperatures (50°C, 56°C and 62°C) and incubation periods (30, 60 and 90 minutes).
**Fig 12****. Bradford protein** assay **of allogeneic PRGF diluted in physiological saline (1:2) (example 4).** Protein quantification (mg/ml) at different temperatures (50°C, 56°C and 62°C) and incubation periods (30, 60 and 90 minutes).

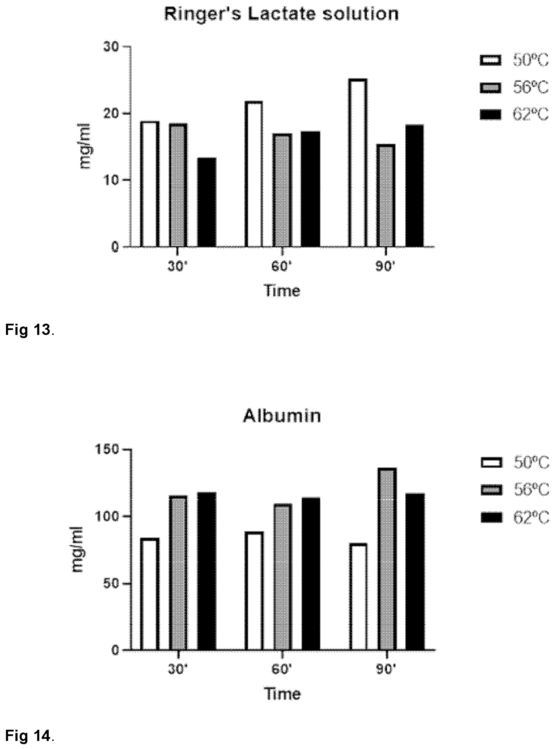

**Fig 13****. Bradford protein assay of allogeneic PRGF diluted in Ringer's Lactate solution (1:2) (example 4).** Protein quantification (mg/ml) at different temperatures (50°C, 56°C and 62°C) and incubation periods (30, 60 and 90 minutes).
**Fig 14****. Bradford protein assay of allogeneic PRGF diluted in human albumin (1:2) (example 4).** Protein quantification (mg/ml) at different temperatures (50°C, 56°C and 62°C) and incubation periods (30, 60 and 90 minutes).

### DETAILED DESCRIPTION OF EMBODIMENTS

The present invention provides a method for obtaining blood derivatives or blood derived products particularly suitable for allogeneic use. In particular, and as shown in the examples, allogeneic blood derivatives or blood derived products can be advantageously obtained by inactivating complement system proteins and removing other components from the blood derivatives or blood derived products which may cause rejection in a receiving organism, thereby reducing the risk of an immune reaction as much as possible. To that end, and as shown in the examples, blood derivatives or blood derived products are centrifuged in the range of from 100g to 6000g, for a time of between 5 and 30 minutes, at a temperature between 4°C and 25°C, and then incubated at a temperature range between 36°C to 72°C for a time range between 15 minutes and 120 minutes. Afterwards, clot remains and cellular and platelet debris from the product resultant from the incubation are preferably removed by preferably carrying out a clarification and/or filtration step of the product.

Therefore, a first aspect of the invention refers to a method for obtaining a composition of blood derivatives or blood derived products, particularly suitable for allogenic use, comprising the steps of:
a) centrifuging the blood derivatives or blood derived products in the range of from 100g to 6000g, for a time of between 5 and 30 minutes, at a temperature between 4°C and 25°C,
b) incubating the product resultant from step (a) at a temperature range between 36°C to 72°C for a time range between 15 minutes and 120 minutes;
c) preferably removing clot remains and cellular and platelet debris from the product resultant from step (b) by preferably carrying out a clarification and/or filtration step of the product resultant from step (b), and
d) optionally collecting the composition resultant from step (c).

In this method, a new blood derivative or blood derived product shall be obtained by inactivating complement proteins and removing compounds which may cause rejection in the receiving organism, thereby reducing the risk of an immune reaction as much as possible. To that end, the blood derivatives or blood derived products are centrifuged in a range of from 100g to 6000g, being preferable from 500g to 2500g, and being more preferable at 700g, during a period of time between 5 and 30 minutes, being preferable between 5 and 20 minutes and more preferable for about 15 minutes, at a temperature between 4°C and 25°C, being preferable a temperature of 20°C. The centrifugation can be used both in fixed-angle rotor, as in the swing-bucket rotor, the latter being preferred for the present invention.

After centrifugation (step (a)), the sample will be incubated, in step a), at a temperature range between 36°C to 72°C for a time range between 15 minutes and 120 minutes. Preferably, step b) is carried out by incubating the product resultant from step (a) at a temperature range between about 50°C to about 62°C for a time range between 30 minutes and 90 minutes. More preferably, step b) is carried out by incubating the product resultant from step (a) at a temperature of about 56°C for a time of about 60 minutes.

As used in the present invention, "blood derivatives or blood derived products" are understood as derivatives of whole blood such as plasma or serum. Most preferably, blood derivatives or blood derived products are platelet derivatives such as platelet-rich plasma (PRP), fibrin glue (FG), platelet gel (PG), plasma rich in growth factors (PRGF), platelet-rich fibrin (PRF), hyperacute serum (HAS), serum eye-drops (E-S), PRP eye-drops (E-PRP) and platelet lysates (PL). It is further noted that platelet-poor plasma (PPP) shall also be understood or included as a blood derivative or blood derived product according to the present invention.

Platelet derivatives, and also products such as PPP, are preferably allogeneic. In this sense, it is noted that the use of autologous platelet derivatives avoids any type of virus or prion contamination and immune reactions associated with allogeneic proteins. Although the volume of autologous platelets may be sufficient for clinical use, limitations of these types of products include wide variability in quality due to changes in platelet counts and GF (growth factors) content that are influenced by the patient's age and biological conditions and also that the administration of autologous platelets is not always possible (i.e., it is not possible in immunodeficient patients, among others). In contrast, allogeneic platelet derivatives are prepared from healthy donor blood using standard working procedures that guarantee products enriched in platelets and GF (growth factors), with minimal contamination from red blood cells and leucocytes than single-donor batches. By using the method of the present invention in allogeneic platelet derivatives we obtained all of the advantages of these types of products avoiding any type of immune reactions associated with allogeneic proteins. That is, by using the method described in the first aspect and inactivating complement proteins and removing compounds from allogeneic platelet derivatives which may cause rejection in the receiving organism, the risk of an immune reaction is avoided.

There are no standardised protocols for the preparation of platelet derivatives in clinical practice: the parameters considered during the preparation include the number and concentration of platelets over baseline, centrifugation conditions and activation of platelets. All these parameters contribute to the composition of platelet derivatives and, ultimately, to their therapeutic effect. The general method to prepare platelet derivatives involves sequential steps: blood such as whole blood is collected with or without an anticoagulant (e.g. in acid-citrate-dextrose tubes), centrifuged to concentrate the platelets, then optionally activated to allow the alpha-granules to release their biological molecules. The platelets are then concentrated according to protocols that include centrifugation steps with different speeds (100-300 g), times (4-20 minutes) and temperatures (12-26°C). The number of platelets in the final product, with the exception of platelet poor plasma (PPP), is four to five times greater than the baseline value; all suspensions of platelets in plasma with a platelet count greater than the baseline count can be identified as PRP or platelet concentrates. To obtain a product with a higher concentration of GF, some protocols produce platelet concentrations up to ten times higher than the baseline value by combining low temperatures, high speeds, and various centrifugation cycles.

For example, in order to produce pure platelet-rich plasma (P-PRP), also known as leucocyte-poor platelet-rich plasma (LP-PRP), the whole blood is collected and centrifuged at low speed to separate the red blood cells - which settle at the bottom of the tube - from white blood cells/platelets and an upper plasma layer, which sediment as an intermediate layer (called the buffy coat) and higher layer, respectively. The upper layer is composed of plasma and a gradient of platelets: poor on the surface, intermediate in the middle and rich near the buffy coat. The upper layer and just the superficial layer of buffy coat are transferred into a sterile tube and then centrifuged at high speed to obtain the P-PRP, which consists of the small volume at the bottom of the tube (about the lower one-third) and is mainly composed of platelets; the resulting supernatant (about the upper two-thirds) constitutes platelet-poor plasma (PPP).

Blood derived products according to the present invention are thus preferably leucoreduced blood products such as LP-PRP, which are currently increasingly being employed to produce blood products with residual WBCs < 5 × 10⁶ per unit (99.9 percent or a log 3 leucoreduction). Clinical data suggests that non-haemolytic febrile transfusion reactions can be prevented by leucodepletion. However, it is noted that although LP-PRP might be prefer in the present invention, other types of PRP products such as LR-PRP (leucocyte-rich platelet-rich plasma) are not excluded of the definition of blood derivatives or blood derived products or Platelet derivatives.

Therefore, in a preferred embodiment of the first aspect of the invention, the blood derivatives or blood derived products are platelet derivatives obtained from isolated blood such as whole blood, bone marrow, menstrual fluid, or umbilical cord blood, collected with or without an anticoagulant (e.g. in acid-citrate-dextrose tubes), centrifuged to concentrate the platelets, then activated to allow the alpha-granules to release their biological molecules, whereas the method comprises the steps of:
a) centrifuging the blood derivatives or blood derived products in the range of from 100g to 6000g, for a time of between 5 and 30 minutes, at a temperature between 4°C and 25°C,
b) incubating the product resultant from step (a) at a temperature range between 36°C to 72°C for a time range between 15 minutes and 120 minutes;
c) removing clot remains and cellular and platelet debris from the product resultant from step (b) by preferably carrying out a clarification and/or filtration step of the product resultant from step (b), and
d) optionally collecting the composition resultant from step (c).

In this manner, a composition of plasma rich in growth factors (PRGF) is obtained, which is particularly suitable for allogenic use.

Preferably, the platelet derivatives are selected from the list consisting of platelet-rich plasma (PRP), fibrin glue (FG), platelet gel (PG), plasma rich in growth factors (PRGF), platelet-rich fibrin (PRF), hyperacute serum (HAS), serum eye-drops (E-S), PRP eye-drops (E-PRP) and platelet lysates (PL).

In a preferred embodiment of the first aspect of the invention, the blood derivatives or blood derived products are platelet-rich plasma (PRP), and the method comprises the steps of:
a) centrifuging the PRP in the range of from 100g to 6000g, for a time of between 5 and 30 minutes, at a temperature between 4°C and 25°C,
b) incubating the product resultant from step (a) at a temperature range between 36°C to 72°C for a time range between 15 minutes and 120 minutes;
c) removing clot remains and cellular and platelet debris from the product resultant from step (b) by preferably carrying out a clarification and/or filtration step of the product resultant from step (b), and
d) optionally collecting the composition resultant from step (c).

In this manner, a composition of plasma rich in growth factors (PRGF) is obtained, which is particularly suitable for allogenic use.

In yet another preferred embodiment of the invention, the blood derivatives or blood derived products are allogeneic or non-allogeneic fibrin. Preferably, allogeneic or non-allogeneic fibrin obtained from PPP. In this sense, and as a non-limiting manner of describing how to obtained fibrin from PPP, it is herein noted that once the PRP fraction is obtained, then such isolated platelet-rich plasma, generally obtained by centrifugation of an anticoagulated sample, is centrifugated again in a range of from 100g to 6000g, being preferable from 500g to 2500g, and being more preferable at 700g, during a period of time between 5 and 30 minutes, preferably between 5 and 20 minutes and more preferably for about 15 minutes, at a temperature between 4°C and 25°C, preferably at a temperature of 20°C, and with a maximum acceleration and medium deceleration. The centrifugation can be used both in fixed-angle rotor, as in the swing-bucket rotor, the latter being preferred for the present invention.

Once centrifugation ends, 2 phases will be obtained in the anticoagulated recipients:
1) Lower phase: this yellow-colored/clear phase contains the PRP fraction.
2) Upper phase: this yellow-colored/clear phase contains the "Platelet poor plasma" (hereinafter referred to as "PPP").

The PPP is then collected and centrifugated in a range of from 100g to 6000g, preferably from 500g to 2500g, and more preferably at **1900g**, during a period of time between 5 and 30 minutes, preferably between 5 and 20 minutes and more preferably for about **10 minutes**, at a temperature between 4°C and 25°C, preferably at a temperature of **20°C**, and with a **medium acceleration** and **maximum deceleration**. The centrifugation can be used both in fixed-angle rotor, as in the swing-bucket rotor, the latter being preferred for the present invention.

Once the PPP is centrifugated, the supernatant is collected in a tube or recipient. This new supernatant obtained is herein defined as initial fibrin. In a preferred embodiment, such collected initial fibrin, is then incubated at a temperature range between 36°C to 72°C for a time range between 15 minutes and 120 minutes according to the present invention or according to any of its preferred embodiments. Preferably, the incubation of the initial fibrin is carried out at a temperature range between about 50°C to about 62°C for a time range between 30 minutes and 90 minutes. More preferably, incubation is performed at a temperature of about 56°C for a time of about 60 minutes.

Therefore, in a preferred embodiment of the first aspect of the invention, the blood derivatives or blood derived products are allogeneic or non-allogeneic fibrin, preferably, allogeneic or non-allogeneic fibrin obtained from PPP, and the method comprises the steps of:
a) centrifuging the fibrin in the range of from 100g to 6000g, for a time of between 5 and 30 minutes, at a temperature between 4°C and 25°C,
b) incubating the product resultant from step (a) at a temperature range between 36°C to 72°C for a time range between 15 minutes and 120 minutes;
c) removing clot remains and cellular and platelet debris from the product resultant from step (b) by preferably carrying out a clarification and/or filtration step of the product resultant from step (b), and
d) optionally collecting the composition resultant from step (c).

As a result of the above method, a type of fibrin is obtained which could be optionally mixed or not, with the PRGF obtained in the preferred embodiments of the first aspect of the invention in a range of PRGF:fibrin from 1:1, 1:2, 1:3 or 1:4, preferably 1:3.

It is noted that any of the platelets derivatives referred to in the present invention to practice the method described in the first aspect or in any of its preferred embodiments, such as platelet-rich plasma (hereinafter referred to as "PRP"), can be obtained by a variety of known sources or samples such as, but not limited to, blood such as whole blood, bone marrow, menstrual fluid, or umbilical cord blood. In particular, any of these sources or samples can, in turn, be selected from:
- Autologous: The sample is obtained from the same patient who will be receiving the product/treatment later.
- Allogeneic: The sample is obtained from a donor who is a subject different from the subject who will be receiving the product/treatment later.
- Heterologous: The sample is obtained from a species other than the species that will be receiving the product/treatment, such as a sample of porcine, canine, ovine, bovine, or equine origin, among others.

The sample will be preferably extracted in anticoagulated recipients such as tubes (i.e. 6-7 ml). Preferably, two anticoagulated recipients such as tubes are needed for obtaining the PRP. However, 5, 6, 7, 8, 9, 10, 11, 12, 13, 14, and up to 15 recipients such as tubes may be needed, with the use of 12 recipients such as tubes being preferred so that there is enough sample to perform the different treatments.

Once the sample is extracted and deposited in anticoagulated recipients or tubes, said samples are centrifuged to obtain the platelet derivatives such as PRP. For this purpose and as exemplified herein, for obtaining PRP the samples are preferably centrifuged at a speed of 200g, for 20 minutes, at a temperature of 20°C (±1°C) and with a maximum acceleration and medium deceleration. A swing-bucket rotor is preferably use in the centrifuge. Once centrifugation ends, 3 clearly differentiated phases will be obtained in the anticoagulated recipients:
1) Upper phase: this yellow-colored/clear phase contains the plasma fraction (platelet-rich plasma (PRP) fraction).
2) Intermediate phase: this clear whitish phase contains a small leukocyte fraction.
3) Lower phase: this red-colored phase contains the blood fraction which contains erythrocytes.

The upper fraction (PRP) is collected, preferably in a tube or recipient without disturbing the intermediate/lower phase. If required, the PRP fractions can be drawn out with a micropipette.

Once the platelet derivative, such as the PRP fraction is obtained, then, the present invention can be carried out by practicing the method described in the first aspect or in any of its preferred embodiments. However, it is noted that such methodology can be optionally further improved by, prior to performing step (a) of the method of the present invention, adding or mixing the platelet derivative, such as the PRP fraction, with a formulation agent (FA) at a ratio Platelet derivative(PD):FA, such as PRP:FA, of from 1:1 to 1:10, wherein the formulation agent is selected from a pharmaceutically acceptable excipient or a pharmaceutically acceptable additive.

As used herein, "pharmaceutically acceptable excipient" (PAE) shall be understood as a preferably inter substance mixed with active substance(s) for consistency, osmotic and chemical stability, protection against protein degradation and facilitate its dosage. They can be considered as PAE substances such as Ringer's Lactate solution or physiological saline, among others.

As used herein, "pharmaceutically acceptable additive" (PAA) shall be understood as inert or non-inert substances that are added to the active substance(s) to facilitate and improve the function of the active substance(s) in the body. They can be considered as PAA, additives such as human serum or human albumin solution, among others.

Therefore, in a preferred embodiment of the first aspect of the invention, the blood derivatives or blood derived products are platelet derivatives obtained from isolated blood such as whole blood, bone marrow, menstrual fluid, or umbilical cord blood, collected with or without an anticoagulant (e.g. in acid-citrate-dextrose tubes), preferably centrifuged to concentrate the platelets, then activated to allow the alpha-granules to release their biological molecules, whereas the method comprises the steps of:
a) centrifuging the blood derivatives or blood derived products in the range of from 100g to 6000g, for a time of between 5 and 30 minutes, at a temperature between 4°C and 25°C,
b) incubating the product resultant from step (a) at a temperature range between 36°C to 72°C for a time range between 15 minutes and 120 minutes;
c) removing clot remains and cellular and platelet debris from the product resultant from step (b) by preferably carrying out a clarification and/or filtration step of the product resultant from step (b), and
d) optionally collecting the composition resultant from step (c);
and wherein, prior to step (a), the platelet derivative, such as isolated platelet-rich plasma (hereinafter referred to as "PRP"), obtained by centrifugation of the anticoagulated sample, is added or mixed with a formulation agent (FA) at a ratio of PRP:FA from 1:1 to 1:10, wherein the formulation agent is selected from a pharmaceutically acceptable excipient or a pharmaceutically acceptable additive as defined above.

In a preferred embodiment of the invention, the platelet derivative, such as isolated platelet-rich plasma (hereinafter referred to as "PRP"), obtained by centrifugation of the anticoagulated sample is added or mixed with a formulation agent (FA) at a ratio of PRP:FA about 1:2, wherein the formulation agent is selected from a pharmaceutically acceptable excipient or a pharmaceutically acceptable additive. It is noted that although a ratio of 1:2 is particularly preferred, other potential ratios such as 1:3, 1:4, 1:5, 1:6, 1:7, 1:8, 1:9, or 1:10 can also be used to improve the stability of the platelet derivative composition.

As used herein, the term "about" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the art. Alternatively, "about" can mean a range of up to 20%, or up to 10%, or up to 5%, or up to 1% of a given value. Where particular values are described in the application and claims, unless otherwise stated the term "about" meaning within an acceptable error range for the particular value should be assumed. More preferably, the term "about" shall mean a range of up to 10% of a given value.

In a preferred embodiment, the FA is preferably selected from the group consisting of physiological saline, Ringer's lactate, phosphate buffered saline, Hanks's solution, Hartmann's solution or glucosaline. Also preferably, the FA is preferably selected from the group consisting of serum (autologous, heterologous, allogeneic) or albumin. Preferably, the FA is selected from the list consisting of physiological saline, Ringer's lactate, phosphate buffered saline, serum and albumin or any combination thereof.

It is noted that, preferably, the platelet derivative, such as the isolated platelet-rich plasma (hereinafter referred to as "PRP") obtained by centrifugation of the anticoagulated sample is added or mixed with a formulation agent (FA), as identified above, at the ratios PRP:FA indicated above, prior to activating the platelet derivative, such as platelet rich plasma (PRP).

That is, a preferred embodiment of the first aspect of the invention, refers to a method (herein referred to as "method with formulation agent of the invention") for obtaining a platelet derivative, preferably plasma rich in growth factors, particularly suitable for allogenic use, comprising the steps of:
a) mixing a platelet derivative, preferably obtained by centrifugation of an anticoagulated sample of blood such as whole blood, bone marrow, menstrual fluid, or umbilical cord blood, with a formulation agent (FA) at a ratio PRP:FA of from 1:1 to 1:10, wherein the formulation agent is selected from a pharmaceutically acceptable excipient or a pharmaceutically acceptable additive;
b) activating the platelet derivative resultant from step (a);
c) centrifuging the product resultant from step (b) in the range of from 100g to 6000g, for a time of between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of the platelet derivative resultant from step (e).

Preferably, the platelet derivatives are selected from the list consisting of platelet-rich plasma (PRP), fibrin glue (FG), platelet gel (PG), plasma rich in growth factors (PRGF), platelet-rich fibrin (PRF), hyperacute serum (HAS), serum eye-drops (E-S), PRP eye-drops (E-PRP) and platelet lysates (PL). More preferably, the platelet derivative is PRP. It is further noted that PPP can be also used in this context.

Another embodiment of the first aspect of the invention, in particular of the method with formulation agent of the invention, refers to a method for obtaining a composition of plasma rich in growth factors, particularly suitable for allogenic use, comprising the steps of:
a) mixing platelet-rich plasma (hereinafter referred to as "PRP") obtained by centrifugation of an anticoagulated sample of blood such as whole blood, bone marrow, menstrual fluid, or umbilical cord blood, with a formulation agent (FA) at a ratio PRP:FA of from 1:1 to 1:10, wherein the formulation agent is selected from a pharmaceutically acceptable excipient or a pharmaceutically acceptable additive;
b) activating the platelet rich plasma (PRP) resultant from step (a);
c) centrifuging the product resultant from step (b) in the range of from 100g to 6000g, for a time of between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

In an embodiment, the activation step of the platelet derivatives according to the method with formulation agent of the inventioncan be carried out by any suitable means known to the skilled person. Preferably, such activation, subsequent to the addition of the FA, can be carried out by the addition of a calcium salt, such as calcium chloride, calcium gluconate, calcium lactate, calcium citrate, carbonate calcium or any combination thereof. The calcium salt can be used at different ranges, the addition of the calcium salt, preferably calcium chloride, being preferable at a final concentration of 22.5mM, although a range of from 0.05mM to 50mM is also within the boundaries of the present invention to activate platelets. Other manners in which platelets can be activated is by the addition of bovine thrombin, autologous thrombin, allogeneic thrombin, recombinant thrombin or any type of thrombin if used at a final concentration of between 0.5U/ml and 5U/ml, with 1U/ml being preferable. A combination of the different calcium salts and thrombin is also possible, preferably when used at the indicated concentrations for each of these compounds.

In another embodiment, a further manner in which platelets can be activated is by photoactivation, through the field of electric pulses, ultraviolet or polychromatic light, for at least 3 minutes, or 5 minutes, or 8 minutes, or 10 minutes, or up to 15 minutes, preferably 10 minutes. This activated PRP solution will be referred to hereinafter as plasma rich in growth factors (PRGF). In this sense, further specific processes for obtaining the PRGF are described in: Role of platelets and fibrin in the healing sequence: an in vivo study of angiogenesis and collagen synthesis. Knighton DR, Hunt TK, Thakral KK, Goodson WH 3rd. Ann Surg. 1982 Oct;196(4):379-88; Platelet-Rich Plasma: Properties and clinical applications. Rick G. Smith, B.S., C.C.P., Craig J. Glassmann, C.C.P., and Mark S. Campbell, B.A., C.C.P. Summer 2007 - Vol.2, No.2; Platelet-rich plasma: evidence to support its use. Marx RE. J Oral Maxillofac Surg. 2004 Apr;62(4):489-96; Platelet-rich plasma: Growth factor enhancement for bone grafts. Marx RE, Carlson ER, Eichstaedt RM, Schimmele SR, Strauss JE, Georgeff KR. Oral Surg Oral Med Oral Pathol Oral Radiol Endod. 1998 Jun;85(6):638-46; Platelet-derived growth factor is a chemoattractant for vascular smooth muscle cells. Grotendorst GR, Chang T, Seppä HE, Kleinman HK, Martin GR. J Cell Physiol. 1982 Nov;113(2):261-6; Identification of platelet proteins separated by twodimensional gel electrophoresis and analyzed by matrix assisted laser desorption/ionization-time of flight-mass spectrometry and detection of tyrosinephosphorylated proteins. Marcus K, Immler D, Sternberger J, Meyer HE. Electrophoresis. 2000 Jul;21(13):2622-36 and in Platelets and inflammation. Klinger MH. Anat Embryol (Berl). 1997 Jul;196(1):1-11.

It is in any case noted that the PRGF obtained from the use of platelet derivatives such as PRP should preferably comprise one or more of the following growth factors: Platelet Factor 4, Heparanase, PDGF-AA (platelet-derived growth factor AA), PDGF-AB (platelet-derived growth factor AB), PDGF-BB (platelet-derived growth factor BB), Vascular Endothelial Growth Factor (VEGF), Hepatocyte Growth Factor (HGF), Epidermal Growth Factor (EGF), Transforming growth factor beta 1 (TGFB1), Transforming growth factor beta 2 (TGFB2), Fibronectin, Thromboxan B2, FACTOR V (factor five), Insulin-like growth factor 1 (IGF1), Leukemia inhibitory factor (LIF), Basic fibroblast growth factor (bFGF) and Acidic fibroblast growth factor.

It is noted that the activation step of the method with formulation agent of the invention, step (b), can be followed by no incubation of the sample or an incubation step of the sample for 5 to 120 minutes to facilitate the previously indicated activation reaction. The incubation can be preferably carried out at room temperature (25°C and 1 atmosphere), or 30°C, but preferably in a range of between 35-38°C.

Preferably, once, the activation step of the method with formulation agent of the invention, step (b),, including the above indicated preferred incubation step, is carried out, the method of the first aspect of the invention proceeds to inactivate the proteins of the complement system. In this step, a new PRGF will be obtained from the PRGF obtained by the previous step by inactivating PRGF complement proteins and removing other compounds which may cause rejection in the receiving organism, thereby reducing the risk of an immune reaction as much as possible. To that end, the PRGF resultant from step (b) is centrifuged in a range of from 100g to 6000g, being preferable from 500g to 2500g, and being more preferable at 700g, during a period of time between 5 and 30 minutes, being preferable between 5 and 20 minutes and more preferable for about 15 minutes, at a temperature between 4°C and 25°C, being preferable a temperature of 20°C. The centrifugation can be used both in fixed-angle rotor, as in the swing-bucket rotor, the latter being preferred for the present invention.

After centrifugation (step (c)), the sample will be incubated, in step d), at a temperature range between 36°C to 72°C for a time range between 15 minutes and 120 minutes. Preferably, step d) is carried out by incubating the product resultant from step (c) at a temperature range between about 50°C to about 62°C for a time range between 30 minutes and 90 minutes. More preferably, step d) is carried out by incubating the product resultant from step (c) at a temperature of about 56°C for a time of about 60 minutes.

Therefore, in another preferred embodiment, the method of the first aspect of the invention, in particular the method with formulation agent of the invention, comprises the following steps:
a) mixing a platelet derivative with a formulation agent (FA) at a ratio PRP:FA of about 1:2, wherein the formulation agent is selected from the list consisting of physiological saline, Ringer's lactate, phosphate buffered saline, Hanks's solution, Hartmann's solution, glucosaline, serum (autologous, heterologous, allogeneic) or albumin;
b) activating the platelet derivative resultant from step (a) by the addition of a calcium salt to the product resultant from step a) such as calcium chloride, calcium gluconate, calcium lactate, calcium citrate, calcium carbonate or any combination thereof at a final concentration of between 0.05mM to 50mM;
c) centrifuging the product resultant from step (b) in the range of from 100g to 6000g, for a time of between 5 and 30 minutes, at a temperature between 4°C and 25°C,
d) incubating the product resultant from step (c) at a temperature range between about 50°C to about 62°C for a time range between about 30 minutes and about 90 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

wherein, preferably, step (b) is carried by adding calcium chloride to the product resultant from step (a) at a final concentration of about 22.5mM; and
wherein, preferably, the formulation agent of step (a) is selected from the list consisting of physiological saline, Ringer's lactate, phosphate buffered saline, serum and albumin, and wherein step (b) is carried by adding calcium chloride to the product resultant from step (a) at a final concentration of about 22.5mM. Preferably, the platelet derivatives are selected from the list consisting of platelet-rich plasma (PRP), platelet-poor plasma (PPP), fibrin glue (FG), platelet gel (PG), plasma rich in growth factors (PRGF), platelet-rich fibrin (PRF), hyperacute serum (HAS), serum eye-drops (E-S), PRP eye-drops (E-PRP) and platelet lysates (PL). More preferably, the platelet derivative is PRP and the product resultant from step e) is a composition comprising plasma rich in growth factors.

In yet another preferred embodiment, the method of the first aspect of the invention, in particular the method with formulation agent of the invention, comprises the following steps:
a) mixing platelet-rich plasma (hereinafter referred to as "PRP") obtained by centrifugation of an anticoagulated sample of blood such as whole blood, bone marrow, menstrual fluid, or umbilical cord blood, with a formulation agent (FA) at a ratio PRP:FA of about 1:2, wherein the formulation agent is selected from the list consisting of physiological saline, Ringer's lactate, phosphate buffered saline, Hanks's solution, Hartmann's solution, glucosaline, serum (autologous, heterologous, allogeneic) or albumin;
b) activating the platelet rich plasma (PRP) resultant from step (a) by the addition of a calcium salt to the product resultant from step a) such as calcium chloride, calcium gluconate, calcium lactate, calcium citrate, calcium carbonate or any combination thereof at a final concentration of between 0.05mM to 50mM;
c) centrifuging the product resultant from step (b) in the range of from 100g to 6000g, for a time of between 5 and 30 minutes, at a temperature between 4°C and 25°C,
d) incubating the product resultant from step (c) at a temperature range between about 50°C to about 62°C for a time range between about 30 minutes and about 90 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

wherein, preferably, step (b) is carried by adding calcium chloride to the product resultant from step (a) at a final concentration of about 22.5mM; and
wherein, preferably, the formulation agent of step (a) is selected from the list consisting of physiological saline, Ringer's lactate, phosphate buffered saline, serum and albumin, and wherein step (b) is carried by adding calcium chloride to the product resultant from step (a) at a final concentration of about 22.5mM.

Importantly, it is noted that in a first alternative first aspect of the invention, steps a) and b) of the method with formulation agent of the invention, may be ordered differently as described below (steps a) and b) can be interchange):
a) activating a platelet rich plasma (PRP) obtained by centrifugation of an anticoagulated sample of whole blood, Bone marrow, Menstrual fluid, or Umbilical cord blood;
b) mixing the product resultant from step (a) with a formulation agent (FA) at a ratio PRP:FA of from 1:1 to 1:10, wherein the formulation agent is selected from a pharmaceutically acceptable excipient or a pharmaceutically acceptable additive;
c) centrifuging the product resultant from step (b) in the range of from 100g to 6000g, for a time of between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

It is further noted that, in a second alternative aspect, the step of stabilizing the sample by adding the FA of the method with formulation agent of the invention may be even performed after the incubation step to inactivate the proteins (see step d) above) and prior to removing clot remains and cellular and platelet debris from the product resultant from this step, if the FA is selected from the list consisting of physiological saline, Ringer's lactate, or phosphate buffered saline.

In a further embodiment of the invention, any of the above indicated methods of the invention can further comprise, preferably after step (e) or (f) in the case of the methods with formulation agent of the invention, the addition of a therapeutic agent such as mesenchymal stem cells (MSCs), preferably MSCs derived from adipose tissue, bone marrow, dental pulp, umbilical cord, amniotic fluid, synovial fluid/membrane, liver, spleen, testicles, menstrual blood, dermis, skeletal muscle, periosteum, lung, and/or peripheral blood, steroids, chondrogenic stimulating factors, monosaccharides, oligosaccharides, polysaccharides, glycosaminoglycans, collagen, essential fatty acids, or trophic factors among others. In a further embodiment of the invention any of the cited biomolecules, cells, trophic factors and different therapeutic agent can be combined between them.

A second aspect of the invention refers to a composition, preferably of plasma rich in growth factors, obtained or obtainable by the method of the first aspect of the invention including any of its alternative aspects.

A third aspect of the invention refers to a composition characterized by comprising:
1. The composition, preferably of plasma rich in growth factors, obtained by the method of the first aspect of the invention including any of its alternative aspects;
2. a therapeutic agent such as mesenchymal stem cells (MSCs), preferably MSCs derived from adipose tissue, bone marrow, dental pulp, umbilical cord, amniotic fluid, synovial fluid/membrane, liver, spleen, testicles, menstrual blood, dermis, skeletal muscle, periosteum, lung, and/or peripheral blood, steroids, chondrogenic stimulating factors, monosaccharides, oligosaccharides, polysaccharides, glycosaminoglycans, collagen, essential fatty acids, or trophic factors; and
3. optionally a pharmaceutically acceptable vehicle or carrier, excipient and further active ingredients.

It is noted that any of the compositions of the second or third aspects of the invention can comprise any one or more of the following: emollients (such as aloe vera), absorbents (such as cellulose), anti-aggregants (such as silicon), anti-dandruff (such as clotrimazole), anti-foaming agents (such as dimethicone), anti-microbial (such as hydrogen peroxide), antioxidant (such as hydroquinone), antiseborrheic (such as Quercus extract), antistatic (such as polyethylene), astringent (such as rosehip oil), binders (such as dextrans), whitener (such as hydroquinone), buffers (such as citric acid), volumizing agents (such as kaolin), chelating agents (such as EDTA), cleansing agent (such as lauric acid), cosmetic colorant (such as riboflavin), denaturant (such as eucalyptus), deodorant (such as perfume), emulsifiers (such as capric acid), emulsion stabilizers (such as pectin), film-forming agents (such as chitosan), gelling agents (such as carbomers), hair conditioners (such as casein), humectants (such as lactic acid), and/or hydrotropes (such as toluene sulfonic acid).

Furthermore, the compositions of the second or third aspects of the invention can be comprised or be in the form of a hydrogel, gel, cream, polymer, fiber/mesh, foam, serum, or base inks for 3D/4D/5D printing. In addition, the compositions of the second or third aspects of the invention can comprise Fibrin

In a further aspect of the present invention, the invention provides methods of treating loss of function and integrity of soft tissue, or in the treatment of an inflammatory skin disorders.

As used herein, the term "loss of function and integrity of soft tissue" shall be understood as reduction of size and/or biological and physiological function of several stromal tissues and cells (herein defined as "connective tissue"). As its name implies, "connective tissue" is a term given to several body tissues that connect, support, and help bind other tissues. Connective tissue comprises several types of fibers (collagen fibers, elastic fibers, reticular fibers) areolar tissue, reticular tissue, adipose tissue, dense connective tissue, dense regular connective tissue,dense irregular connective tissue, cartilage, hyaline cartilage, fibrocartilage, elastic cartilage, bones, muscles.

In addition, for use in therapy and methods of treatment, the cell suspensions of the invention will be delivered to the subject in a therapeutically effective amount.

The purpose of the following examples is merely to illustrate the present invention.

### EXAMPLES

### 1. EXAMPLE 1.

### METHODOLOGY

Once received, the blood sample is introduced in the Grade C room. All the handling operations of ACD-B-anticoagulated tubes and the subsequent steps of the different handling operations performed with the phases or compositions obtained were carried out in a laminar flow cabinet (Safe 2020 laminar flow cabinet).

The ACD-B-anticoagulated tubes were centrifuged in the Grade C room in a Sorvall 16R centrifuge at a speed of **200xg, for 20 minutes, at a temperature of 20°C (±1°C) and with an acceleration of 9 and a deceleration of 5.** A swing-bucket rotor was used in the centrifuge.

Once the centrifugation ended, **3 clearly differentiated phases were obtained in the ACD-B-anticoagulated tube:**
- **Upper phase:** this **yellow-colored/clear** phase contains the plasma fraction **(platelet-rich plasma (PRP) fraction).**
- **Intermediate phase:** this clear whitish phase contains a small leukocyte fraction.
- **Lower phase:** this red-colored phase contains the blood fraction which contains erythrocytes.

**The upper fraction (PRP) was collected in a 50 ml Falcon tube** without disturbing the intermediate/lower phase. If required, the PRP fractions could be drawn out by using a micropipette.

Once the entire PRP fraction was collected in the 50 ml Falcon tube, **an adjuvant was added** (in this experiment, 5% human serum was added). 45 µl/ml of CaCl₂ were added for platelet activation, incubating at 37°C for 90 minutes. After incubation, the volume of activated PRP (PRGF) was split into two tubes:
1) The first tube was treated as an "autologous PRGF", being purified directly (high-speed centrifugation, 9000g, 10 minutes, 20°C, and maximum acceleration and deceleration). The PRGF supernatant is then filtered through a 0.22 µm filter and "autologous" aliquots are completed.
2) The second tube was treated as an "allogeneic PRGF", first being centrifuged at 1000g for 10 minutes at 20°C with a maximum acceleration and deceleration and then incubated at 56°C for 90 minutes. After incubation, it was purified under the same conditions as the "autologous PRGF" and filtered with the same type of filter to obtain "allogeneic PRGF" aliquots.

### RESULTS

### COOMBS TEST

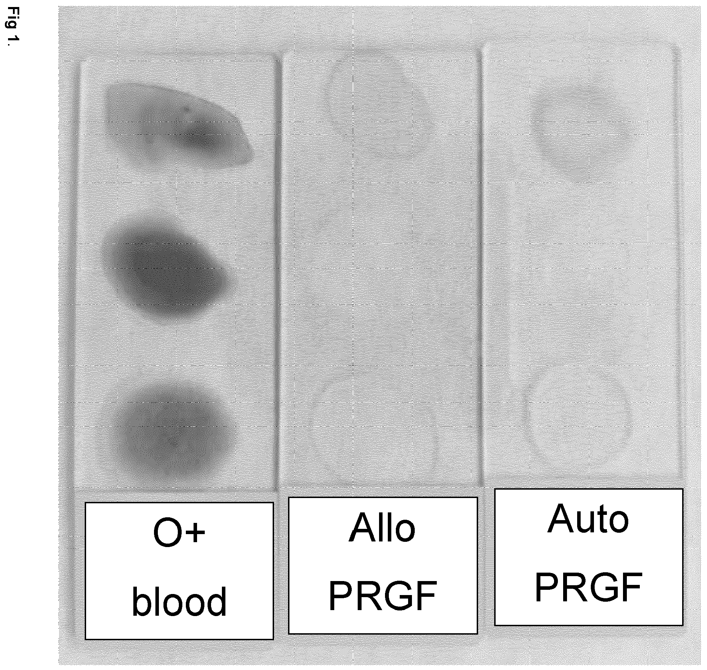

In the Coombs test (see figure 1), the donor's blood group (O+) is confirmed by observing agglutination of only the antigen corresponding to the Rh group, which confirms the effectiveness of the test. No reaction in any antigen is observed in the "autologous PRGF" sample or in the "allogeneic PRGF" sample, so the presence of antigens corresponding to the blood group is ruled out in both cases.

### PROTEIN QUANTIFICATION

The total protein concentration in the allogeneic serum, autologous PRGF, and allogeneic PRGF was quantified, obtaining the following results:

| **Autologous PRGF** | **Allogeneic PRGF** | **Allogeneic Serum** |
|---|---|---|
| 68.00 mg/ml | 59.40 mg/ml | 108.46 mg/ml |

It can be observed that, despite the additional centrifugations and incubation at 56°C for 90 minutes, protein concentrations in both types of PRGF are very similar, so it could be deduced that protein concentration is not affected during the complement inactivation process.

### CH50 TEST

| **Autologous PRGF (C); Allogeneic PRGF (A)** | **Standard values** | **Obtained values** |
|---|---|---|
| **C** | [31.6-57.6] U/ml | 48.0 U/ml |
| **A** | | 10.3 U/ml |

The CH50 test is used to determine the amount and activity of complement system proteins (measured in U/ml). Taking the standard values of complement system proteins as a reference (31.6-57.6 U/ml), measurements are performed on the "autologous PRGF" control and "allogeneic PRGF" samples. It is observed in the first case that the values are within the established range (48 U/ml). However, in the second case, the activity of the complement proteins drops to 10.3 U/ml, **which confirms that incubation at 56°C led to the desired effect of significantly reducing the activity of the complement system.**

### EXAMPLE 2.

### METHODOLOGY

Once received, the blood sample is introduced in a laminar flow cabinet. All the handling operations of anticoagulated tubes and the subsequent steps of the different handling operations performed with the phases or compositions obtained were carried out in a laminar flow cabinet.

The anticoagulated tubes were centrifuged at a speed of **200g, for 20 minutes, at a temperature of 20°C (±1°C) and with a maximum acceleration and medium deceleration.** A swing-bucket rotor was used in the centrifuge.

Once centrifugation ends, **3 clearly differentiated phases were obtained in the ACD-B-anticoagulated tube:**
**Upper phase:** this **yellow-colored/clear** phase contains the plasma fraction **(platelet-rich plasma (PRP) fraction)**
**Intermediate phase:** this clear whitish phase contains a small leukocyte fraction.
**Lower phase:** this red-colored phase contains the blood fraction which contains erythrocytes.

**The upper fraction (PRP) was collected in a tube or recipient** without disturbing the intermediate/lower phase.

Once the entire PRP fraction was collected in the tube or recipient, **the adjuvant was added** (in this experiment, 5% human serum was added). 45 µl/ml of CaCl₂ were added for platelet activation, incubating at 37°C for 90 minutes. After incubation, the volume of activated PRP (PRGF) was split into two tubes:
1) The first tube was treated as an "autologous PRGF", being purified directly (high-speed centrifugation, 9000g, 10 minutes, 20°C, and maximum acceleration and deceleration). The PRGF supernatant is then filtered through a 0.22 µm filter and "autologous PRGF" aliquots are completed.
2) The second tube was treated as an "allogeneic PRGF", first being centrifuged at 1000g for 10 minutes at 20°C with a maximum acceleration and deceleration then incubated at 56°C for 90 minutes. After incubation, it was purified under the same conditions as the "autologous PRGF" and filtered with the same type of filter to obtain "allogeneic PRGF" aliquots.

### RESULTS

### COOMBS TEST

### PROTEIN QUANTIFICATION

The total protein concentration in the allogeneic serum, autologous PRGF, and allogeneic PRGF was quantified, obtaining the following results:

| **Autologous PRGF** | **Allogeneic PRGF** | **Allogeneic Serum** |
|---|---|---|
| 61.73 mg/ml | 78.67 mg/ml | 156.58 mg/ml |

Like in example 1, it can be observed that the protein concentration does not decrease due to the effect of incubation at 56°C. However, in this case, an increase in protein concentration is observed in the "allogeneic PRGF" with respect to the "autologous PRGF".

### CH50 TEST

| **Autologous PRGF (C); Allogeneic PRGF (A)** | **Standard values** | **Obtained values** |
|---|---|---|
| **C** | [31.6-57.6] U/ml | > 60.0 U/ml |
| **A** | | < 10.0 U/ml |

Like in example 1, the CH50 test is performed to determine the levels and activity of complement system proteins. Using standard values as a reference, it can be seen that the values are above 60 U/ml in the "autologous PRGF" control sample, whereas in the "allogeneic PRGF" sample, the values are below 10 U/ml.

### EXAMPLE 3.

### METHODOLOGY

Once received, the blood sample is introduced in a laminar flow cabinet. All the handling operations of anticoagulated tubes and the subsequent steps of the different handling operations performed with the phases or compositions obtained were carried out in a laminar flow cabinet.

The anticoagulated tubes were centrifuged at a speed of **200g, for 20 minutes, at a temperature of 20°C (±1°C) and with a maximum acceleration and medium deceleration.** A swing-bucket rotor was used in the centrifuge.

Once centrifugation ended, **3 clearly differentiated phases were obtained in the ACD-B-anticoagulated tube:**
- **Upper phase:** this **yellow-colored/clear** phase contains the plasma fraction **(platelet-rich plasma (PRP) fraction).**
- **Intermediate phase:** this clear whitish phase contains a small leukocyte fraction.
- **Lower phase:** this red-colored phase contains the blood fraction which contains erythrocytes.

**The upper fraction (PRP) was collected in a tube or recipient** without disturbing the intermediate/lower phase.

Once the entire PRP fraction was collected in the tube or recipient, **the adjuvant was added** (in this experiment, 5% human serum was added). 45 µl/ml of CaCl₂ were added for platelet activation, incubating at 37°C for 90 minutes. After incubation, the volume of activated PRP (PRGF) was split into two tubes:
1) The first tube was treated as an "autologous PRGF", being purified directly (high-speed centrifugation, 9000g, 10 minutes, 20°C, and maximum acceleration and deceleration). The PRGF supernatant was then filtered through a 0.22 µm filter and "autologous PRGF" aliquots are completed.
2) The second tube was treated as an "allogeneic PRGF", first being centrifuged at 1000g for 10 minutes at 20°C with a maximum acceleration and deceleration and then incubated at 56°C for 90 minutes. After incubation, it was purified under the same conditions as the "autologous PRGF" and filtered with the same type of filter to obtain "allogeneic PRGF" aliquots.

### RESULTS

### COOMBS TEST

### PROTEIN QUANTIFICATION

The total protein concentration in the allogeneic serum, autologous PRGF, and allogeneic PRGF was quantified, obtaining the following results:

| **Autologous PRGF** | **Allogeneic PRGF** | **Allogeneic Serum** |
|---|---|---|
| 53.51 mg/ml | 72.95 mg/ml | 100.85 mg/ml |

Like in example 1, it can be observed that the protein concentration does not decrease due to the effect of incubation at 56°C. However, in this case, an increase in protein concentration is observed in the "allogeneic PRGF" with respect to the "autologous PRGF".

### CH50 TEST

| **Autologous PRGF (C); Allogeneic PRGF (A)** | **Standard values** | **Obtained values** |
|---|---|---|
| **C** | [31.6-57.6] U/ml | > 60.0 U/ml |
| **A** | | < 10.0 U/ml |

Like in examples 1 and 2, the CH50 test was performed to determine the levels and activity of complement system proteins. Using standard values as a reference, it can be seen that the values are above 60 U/ml in the "autologous PRGF" control sample, whereas in the "allogeneic PRGF" sample, the values are below 10 U/ml, which cannot be quantified by means of the test.

### EXAMPLE 4

After obtaining the results of the three preceding experiments, the inventors decided to use other adjuvants, besides human serum, as well as new temperature conditions and incubation times, to define whether the already observed considerable reduction in immunogenicity could be more accurately defined. Moreover, the inventors also sought to thereby optimize the formulation of the product in search of the ideal adjuvant and concentration that will also allow optimizing final protein concentration and protein stability.

### METHODOLOGY

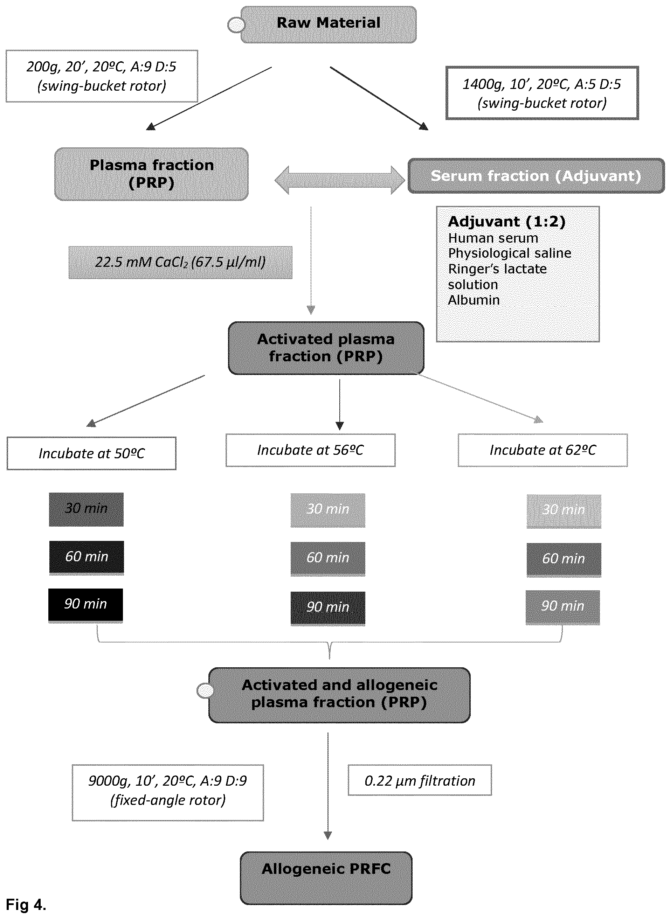

A chart illustrating the manufacture of the allogeneic PRGF used for conducting the entire experiment is provided at figure 4.

First, peripheral blood (raw material) was obtained from a healthy A⁻ donor. The blood sample was collected in 13 anticoagulated tubes and 5 serum separator tubes.

The anticoagulated tubes were centrifuged at 200g for 20 minutes at a temperature of 20°C and with a maximum acceleration and medium deceleration using a swing-bucket rotor, where three clearly differentiated phases were obtained, from which only the yellow-colored upper phase containing the plasma fraction (PRP) was collected.

The serum separator tubes were centrifuged at 1400g for 10 minutes at a temperature of 20°C and with a medium acceleration and deceleration using a swing-bucket rotor, where two phases are obtained, from which the upper phase containing serum was collected.

After collecting the serum and PRP fractions, the corresponding formulation agent was added at a PRP:FA proportion of 1:2. The following adjuvants were studied:
Human serum (HS)
Physiological saline (SF)
Ringer's lactate solution (RL)
Human albumin 200g/l (A)

In each condition, 6 ml of plasma were mixed with 12 ml of FA, obtaining a total volume of 18 ml.

After homogenizing the samples, platelet activation was performed by adding calcium chloride to the PRP+adjuvant mixture. To that end, **67.5µl of CaCl₂/ml** of FA were added to each sample, so 810 µl of CaCl₂ were added for each case.

Then, allogeneic PRGF was obtained by inactivating complement system proteins and removing other components of the PRGF which may cause rejection in the receiving organism, thereby reducing the risk of an immune reaction as much as possible.

To that end, the PRGF was centrifuged at 1000g for 10 minutes at a temperature of 20°C and with a maximum acceleration and a deceleration.

After centrifugation, the samples were incubated at **3 different temperatures (50, 56, and 62°C)** and for **3 times (30 minutes, 60 minutes, and 90 minutes).**

After obtaining the allogeneic sample, a high-speed centrifugation (9000g, at a temperature of 20°C, for 10 minutes, and with a maximum acceleration and deceleration, with a fixed-angle rotor) was performed to purify the sample.

Finally, the composition was subjected to sterilizing filtration with a 0.22 µm hydrophilic filter.

Each study condition included 1.5-2.0 ml of allogeneic PRGF to allow conducting all the selected studies: Coombs reactivity test, total protein quantification, CH50, CD4⁺/CD8⁺ cytometry, and proteomics study.

### RESULTS

### COOMBS TEST

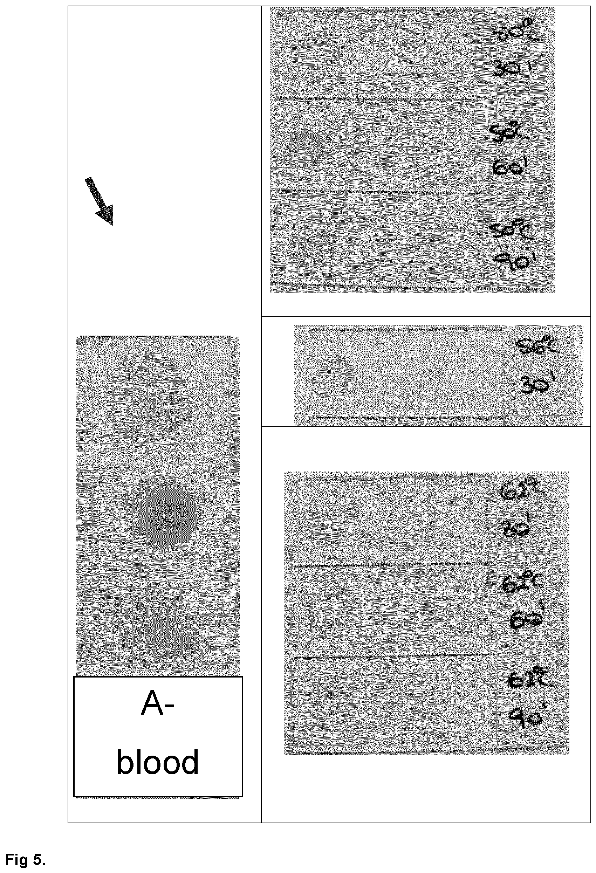

The Coombs reactivity test was conducted for all the study samples. Blood from the A⁻ donor was used as a positive control. The images in figure 5 show the results.

The blue-colored halo corresponds to the group A antigen, the orange-yellow-colored halo corresponds to the group B antigen, and the white-colored halo corresponds to antigen D which corresponds to Rh.

### PROTEIN QUANTIFICATION

The following tables show the results obtained in protein quantification by means of the Bradford method. The results are shown in mg/ml of total protein.

In the case of human serum (HS), the highest protein concentration was obtained in the condition of 56°C and a 60-minute incubation. However, the value of 48.85 mg/ml, which was obtained at 62°C with a 30-minute incubation, is also close.

| HUMAN SERUM (HS) | | | |
|---|---|---|---|
| **T** | **50°C** | **56°C** | **62°C** |
| **30 min** | Vt: 40.39 | Vt: 43.59 | Vt: 48.85 |
| **60 min** | Vt: 35.52 | Vt: 49.68 | Vt: 33.27 |
| **90 min** | Vt: 40.65 | Vt: 41.03 | Vt: 46.62 |

With physiological saline (SF), total protein values decreased considerably. In this case, the highest values were obtained at a temperature of 50°C with incubation times of 60 and 90 minutes.

| PHYSIOLOGICAL SALINE (SF) | | | |
|---|---|---|---|
| **T** | **50°C** | **56°C** | **62°C** |
| **30 min** | Vt: 19.90 | Vt: 15.70 | Vt: 19.61 |
| **60 min** | Vt: 21.24 | Vt: 18.39 | Vt: 15.27 |
| **90 min** | Vt: 21.83 | Vt: 16.88 | Vt: 19.02 |

Observations similar to those seen for physiological saline apply to Ringer's lactate solution (RL). The highest protein concentration was obtained in the 90-minute incubation at 50°C, followed by the 1-hour incubation at the same temperature.

| RINGER'S LACTATE SOLUTION (RL) | | | |
|---|---|---|---|
| **T** | **50°C** | **56°C** | **62°C** |
| **30 min** | Vt: 18.91 | Vt: 18.46 | Vt: 13.40 |
| **60 min** | Vt: 21.85 | Vt: 17.14 | Vt: 17.42 |
| **90 min** | Vt: 25.22 | Vt: 15.43 | Vt: 18.33 |

In the case of albumin (A), the highest protein concentration was obtained in the condition of 56°C and a 90-minute incubation. However, the mean values of 56°C and 62°C are similar (120.39 mg/ml in the condition of 56°C and 116.10 mg/ml in the condition of 62°C).

| ALBUMIN (A) | | | |
|---|---|---|---|
| **T** | **50°C** | **56°C** | **62°C** |
| **30 min** | Vt: 84.35 | Vt: 115.36 | Vt: 117.75 |
| **60 min** | Vt: 89.21 | Vt: 109.81 | Vt: 113.56 |
| **90 min** | Vt: 79.97 | Vt: 136.01 | Vt: 117.01 |

### CH50 TEST

The following table shows the results obtained in the CH50 test for the complement system by means of immunoturbidimetry. The results are shown in U/ml and the reference values are between 31.6 U/ml and 57.6 U/ml.

It should be noted that only human serum (SA) serum samples were analyzed as they were considered the worst case.

| HUMAN SERUM (SA) | | | |
|---|---|---|---|
| **T** | **50°C** | **56°C** | **62°C** |
| **30 min** | 12.0 | 10.2 | 10.2 |
| **60 min** | 11.4 | <10.0 | 10.5 |
| **90 min** | 11.0 | 10.8 | <10.0 |

As can be seen in the table above, all the analyzed batches have values that are far below the lower reference limit established by the external laboratory. The lowest values were found in batches incubated at 56°C for 60 minutes and incubated at 62°C for 90 minutes.

### CD4+/CD8+ CYTOMETRY

Flow cytometry is performed on autologous and allogeneic PRGF samples to verify whether helper T lymphocytes (CD4+) and cytotoxic T lymphocytes (CD8+) are present after filtration.

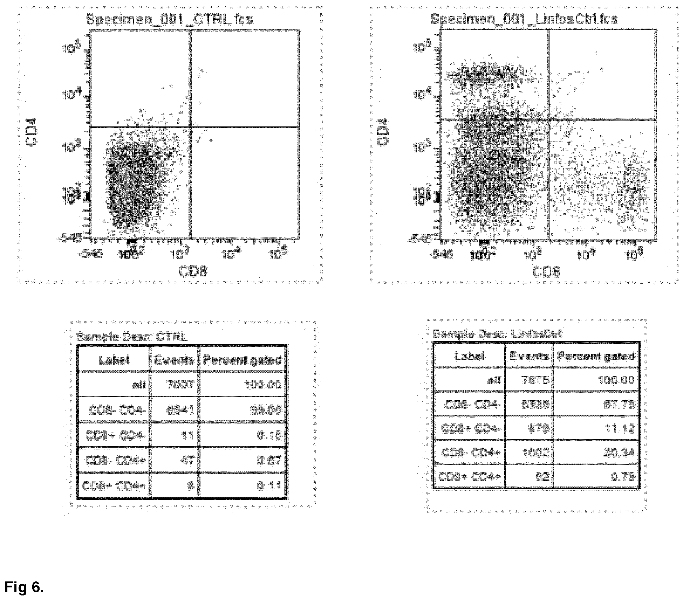

To that end, the CD4+ and CD8+ populations of autologous PRGF and allogeneic PRGF samples from human serum (SA, 56°C, 60 min) were analyzed using a positive control sample extracted from the leukocyte interphase, a fraction of about 1% which contains several types of mononuclear cells including, among them, the lymphocytes of interest (see figure 6).

The CD4+ and CD8+ populations in the positive control sample (*LinfosCtrl*) and in the unstained control *(CTRL)* are analyzed. The CD4+ and CD8+ populations in the *LinfosCtrl* sample are established based on the reference cell population defined in the control sample, observing 11.12% of CD8+ cells and 20.34% CD4+ cells.

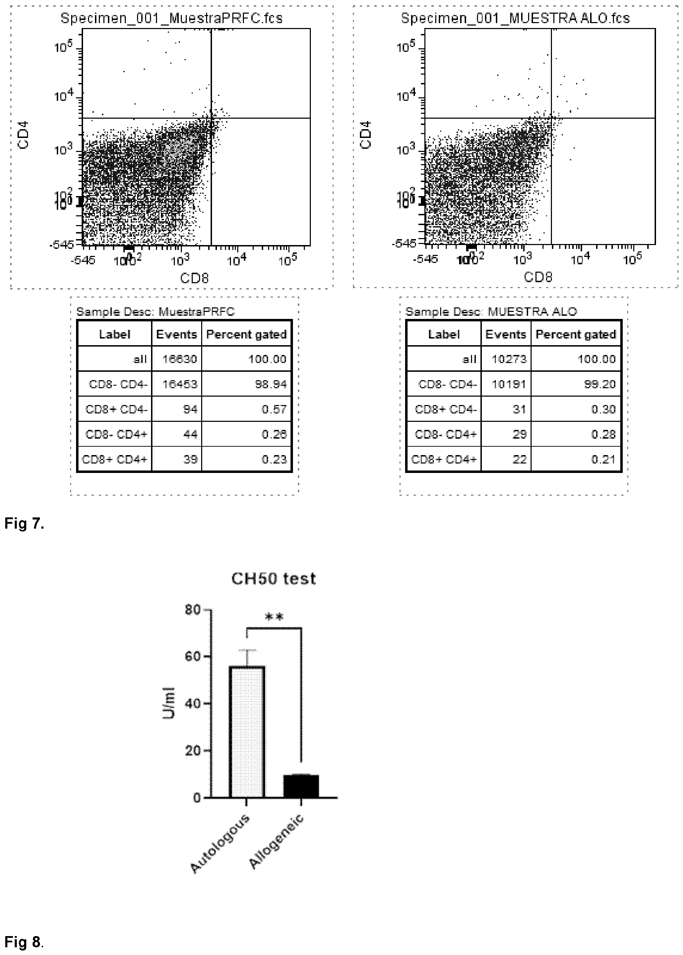

Once the CD4+ and CD8+ populations in the control samples are established, whether the populations of interest are present in the autologous and allogeneic PRGF samples, respectively, is analyzed:
As can be seen in the plots indicated in figure 7, no CD4+ or CD8+ populations are observed, so **the presence of helper T lymphocytes** and cytotoxic lymphocytes is **not observed** in the PRGF samples. Additionally, no defined cell population is observed.

| **Marker** | **Control** | **Autologous** | **Allogeneic** |
|---|---|---|---|
| **CD4+** | 20.34% | 0.26% | 0.28% |
| **CD8+** | 11.12% | 0.57% | 0.30% |
| **CD4+CD8+** | 0.79% | 0.23% | 0.21% |

### PROTEOMICS

The proteomics study allows identifying and classifying each and every protein present in the solution. This analysis allows generating a large amount of information about different **proteins with biological activity in the product,** where the main biological functions of the PRGF manufactured herein can be accurately defined and the **treatments provided by Regenia can thus be optimized as much as possible.**

Preliminary data has confirmed the following information about proteins found in the PRGF solution manufactured herein:
A. **Proteins with immunomodulatory activity** (immunoglobulins and complement proteins)
B. Proteins involved in **metal adsorption and transport** (transferrins, ceruloplasmins, etc.)
C. **High-abundance serum proteins,** such as albumin or transferrins
D. **Growth factors** which stimulate **cell proliferation** (IGF, EGF, TGF-β, etc.)
E. **Proteins with lipid, carbohydrate, and protein metabolism regulatory activity** (peroxidases, esterases, transaminases, kinases, etc.)
F. Proteins involved in **cell adhesion and endothelial integrity** (cadherins, fibronectin, lectins)
G. Proteins **involved in the absorption and transport of different vitamins** (vitamin K, vitamin D, etc.)
H. Proteins involved in **homeostasis and coagulation regulation** (coagulation factor IX, coagulation factor VIII, heparin, thrombins, etc.)
I. Proteins with **antioxidant, antibacterial, and anti-inflammatory** activity (haptoglobins, platelet basic protein, etc.)

### CONCLUSIONS OF EXAMPLE 4

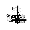 In allogeneic PRGF samples, no antigenic or Rh factor reaction is observed, which confirms the compatibility of PRGF samples in different blood groups.
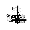 Total protein was quantified by means of the Bradford method, with the highest protein concentrations being observed in PRGF samples with albumin and human serum, respectively. Protein values in the physiological saline and Ringer's lactate solution conditions are lower than in the other two conditions, a result which can be expected taking into account that the other two FA solutions contain protein therein.
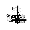 It can be observed that the fluctuation in the protein concentration of PRGF samples with albumin and human serum is greater than PRGF samples with physiological saline and Ringer's lactate solution.
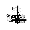 In the PRGF samples with human serum (considered the worst case as it has human serum as the FA) delivered for complement system analysis, it can be seen that the U/ml values are far below the reference values in all the analyzed cases (<12 U/ml in all the cases when the reference values are 31.6 U/ml-57.6 U/ml). The values obtained at temperatures of 56°C and 62°C are lower than at 50°C, which indicates the positive effect of temperature on complement system degradation.
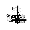 The presence of CD4+ and CD8+ T lymphocytes in allogeneic PRGF samples was analyzed, with the results demonstrating the absence of lymphocytes in the studied cases as compared to control peripheral blood lymphocyte samples.

## Claims

1. A method of obtaining a composition of plasma rich in growth factors, comprising the steps of:
a) mixing platelet-rich plasma (hereinafter referred to as "PRP") obtained by centrifugation of an anticoagulated sample of whole blood, bone marrow, menstrual fluid, or umbilical cord blood, with a formulation agent (FA) at a ratio PRP:FA of from 1:1 to 1:10, wherein the formulation agent is selected from a pharmaceutically acceptable excipient or a pharmaceutically acceptable additive;
b) activating the platelet rich plasma (PRP) resultant from step (a);
c) centrifuging the product resultant from step (b) in the range of from 100g to 6000g, for a time of between 5 and 30 minutes, at a temperature between 4°C and 25°C,
d) incubating the product resultant from step (c) at a temperature range between 36°C to 72°C for a time range between 15 minutes and 120 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

2. The method according to claim 1, wherein step d) is carried out by incubating the product resultant from step (c) at a temperature range between about 50°C to about 62°C for a time range between 30 minutes and 90 minutes.

3. The method according to claim 1 or 2, wherein step c) is performed by centrifuging the product resultant from step (b) in the range of from about 500g to about 2500g, for a time of between 5 and 20 minutes, at a temperature between 15°C and 25°C.

4. The method of any of claims 1 to 3, wherein the FA is a pharmaceutically acceptable excipient selected from the group consisting of physiological saline, Ringer's lactate, phosphate buffered saline, Hanks's solution, Hartmann's solution or glucosaline.

5. The method of any of claims 1 to 3, wherein the FA is a pharmaceutically acceptable additive selected from the group consisting of serum (autologous, heterologous, allogeneic) or albumin.

6. The method of any one of claims 1 to 5, wherein the activation of the activation of the platelet rich plasma (PRP) is carried out by the addition of a calcium salt to the product resultant from step a) such as calcium chloride, calcium gluconate, calcium lactate, calcium citrate, calcium carbonate or any combination thereof.

7. The method of claim 6, wherein the calcium salt shall be added to the product resultant from step a) at a final concentration of between 0.05mM to 50mM.

8. The method according to claim 1, wherein the method comprises the following steps:
a) mixing platelet-rich plasma (hereinafter referred to as "PRP") obtained by centrifugation of an anticoagulated sample of whole blood, Bone marrow, Menstrual fluid, or Umbilical cord blood, with a formulation agent (FA) at a ratio PRP:FA of about 1:2, wherein the formulation agent is selected from the list consisting of physiological saline, Ringer's lactate, phosphate buffered saline, Hanks's solution, Hartmann's solution, glucosaline, serum (autologous, heterologous, allogeneic) or albumin;
b) activating the platelet rich plasma (PRP) resultant from step (a) by the addition of a calcium salt to the product resultant from step a) such as calcium chloride, calcium gluconate, calcium lactate, calcium citrate, calcium carbonate or any combination thereof at a final concentration of between 0.05mM to 50mM;
c) centrifuging the product resultant from step (b) in the range of from 100g to 6000g, for a time of between 5 and 30 minutes, at a temperature between 4°C and 25°C,
d) incubating the product resultant from step (c) at a temperature range between about 50°C to about 62°C for a time range between about 30 minutes and about 90 minutes;
e) removing clot remains and cellular and platelet debris from the product resultant from step (d) by preferably carrying out a clarification and/or filtration step of the product resultant from step (d), and
f) optionally collecting the composition of plasma rich in growth factors resultant from step (e).

9. The method of claim 8, wherein step (b) is carried by adding calcium chloride to the product resultant from step (a) at a final concentration of about 22.5mM.

10. The method of claim 8, wherein the formulation agent of step (a) is selected from the list consisting of physiological saline, Ringer's lactate, phosphate buffered saline, serum and albumin, and wherein step (b) is carried by adding calcium chloride to the product resultant from step (a) at a final concentration of about 22.5mM.

11. The method of any one of claims 1 to 10, wherein the method further comprises, preferably after step (e) or (f), the addition of a therapeutic agent such as mesenchymal stem cells (MSCs), preferably MSCs derived from adipose tissue, bone marrow, dental pulp, umbilical cord, amniotic fluid, synovial fluid/membrane, liver, spleen, testicles, menstrual blood, dermis, skeletal muscle, periosteum, lung, and/or peripheral blood, steroids, chondrogenic stimulating factors, monosaccharides, oligosaccharides, polysaccharides, glycosaminoglycans, collagen, essential fatty acids, or trophic factors.

12. A composition of plasma rich in growth factors obtained or obtainable by the method of any one of claims 1 to 11.

13. A composition, preferably a pharmaceutical composition, comprising the composition of plasma rich in growth factors of claim 12 and further comprising any one or more of the following emollients (such as aloe vera), absorbents (such as cellulose), anti-aggregants (such as silicon), anti-dandruff (such as clotrimazole), anti-foaming agents (such as dimethicone), anti-microbial (such as hydrogen peroxide), antioxidant (such as hydroquinone), antiseborrheic (such as Quercus extract), antistatic (such as polyethylene), astringent (such as rosehip oil), binders (such as dextrans), whitener (such as hydroquinone), buffers (such as citric acid), volumizing agents (such as kaolin), chelating agents (such as EDTA), cleansing agent (such as lauric acid), cosmetic colorant (such as riboflavin), denaturant (such as eucalyptus), deodorant (such as perfume), emulsifiers (such as capric acid), emulsion stabilizers (such as pectin), film-forming agents (such as chitosan), gelling agents (such as carbomers), hair conditioners (such as casein), humectants (such as lactic acid), and/or hydrotropes (such as toluene sulfonic acid).

14. The composition of plasma rich in growth factors of claim 12 or the composition of claim 13, wherein any of the said compositions are comprised or in the form of a hydrogel, gel, cream, polymer, fiber/mesh, foam, serum, or base inks for 3D/4D/5D printing.

15. In addition, the compositions of the second or third aspects of the invention can comprise Fibrin allogeneic or non allogeneic, preferably allogeneic.

16. The composition of plasma rich in growth factors of claim 12 or 14, or the composition of claim 13 for use in the treatment of alopecia or in the treatment of an inflammatory skin disorder.
